# EUROPEAN PATENT SPECIFICATION

(11) **EP 3 168 520 B1**
(45) Date of publication and mention of the grant of the patent: **02.12.2020**
(21) Application number: 14891475.7
(22) Date of filing: 01.08.2014
(51) Int. Cl.: F16M 11/08, F16M 13/02, F16C 19/16, A61B 90/50

(54) **MEDICAL OVERHEAD MAST ARM SYSTEM AND ROTATION SHAFT USED FOR SAME**
MEDIZINISCHES ÜBERKOPFMASTARMSYSTEM UND DREHWELLE DAFÜR
SYSTÈME DE BRAS DE MÂT MÉDICAL SUSPENDU ET AXE DE ROTATION UTILISÉ POUR CELUI-CI

(30) Priority: 06.05.2014 CN 201410187994
(43) Date of publication of application: 17.05.2017
(73) Proprietor: Maquet (Suzhou) Co. Ltd., Suzhou, Jiangsu 215024 (CN)
(72) Inventor: TAO, Xiaowang, Suzhou Jiangsu 215024 (CN); LI, Qunhua, Suzhou Jiangsu 215024 (CN); JI, Ming, Suzhou Jiangsu 215024 (CN)
(74) Representative: WSL Patentanwälte Partnerschaft mbB
(86) International application number: PCT/CN2014/083522
(87) International publication number: WO 2015/168995

(56) References cited:
- EP-A1- 1 473 473
- WO-A1-99/50587
- CN-A- 102 182 759
- CN-A- 102 312 946
- CN-A- 102 312 946
- CN-A- 103 353 050
- CN-A- 103 953 837
- CN-U- 202 211 748
- CN-U- 203 880 364
- US-A1- 2004 104 328
- US-A1- 2005 282 673
- US-A1- 2010 104 234
- US-A1- 2013 221 183
- US-B1- 7 770 860

## Description

### Technical field

The present invention relates to the technical field of medical equipment, and more particularly to a medical pendant arm system and rotation shaft used for the same.

### Background art

Medical pendant system is an indispensable medical equipment used in modern hospital operating room, intensive care unit and the like. A medical pendant arm system is an important component of a medical pendant.

The existing medical pendant arm system mainly has the following drawbacks: incompact overall structure, complicated assembly and long assembly time, relatively more parts, and incapability of installation of electromagnetic brake.

US 7,770,860 B1 relates to a modular ceiling-mounted medical services system including a vertical column supported by an articulating arm assembly. Rotational joints connect the column to the lower arm, the lower arm to the upper arm, and the upper arm to the ceiling mount. Rotational movement at each of the joints is controlled by an electrically-released electromagnetic brake, and all of the brakes may be controlled by a switch panel on the column. Thus, the entire assembly is held in place until the switch is activated.

WO 99/50587 A1 relates to an apparatus for supporting a service column from a ceiling. The apparatus includes a mounting assembly, an arm assembly, and a leveling mechanism. The mounting assembly is adapted to be coupled to the ceiling and includes a tube configured to extend downwardly with respect to the ceiling. The arm assembly includes a first arm coupled to the tube and extending therefrom. A portion of the arm assembly is adapted to be coupled to the service column so that the service column is supportable at a location spaced from the mounting assembly.

CN 102 312 946 A relates to an electromagnetic braking device for a medical tower crane joint. The electromagnetic braking device is arranged at a predetermined distance between a movable joint sleeve and a fixed joint seat; the movable joint sleeve, the fixed joint seat and the electromagnetic braking device are sequentially connected to form a rotatable structure and a joint electromagnetic braking device. The joint electromagnetic braking device is arranged at position of an upper joint or a middle joint of a medical tower crane.

### Summary of invention

One of the purposes of the present invention is to overcome the above-mentioned disadvantages of the existing medical pendant arm system, there is provided a medical pendant arm system, which has compact overall structure, simple assembly and short assembly time, and relatively less parts, and an electromagnetic brake or an air-bag brake can be selectively installed.

The invention is as defined by independent claim 1. Dependent claims disclose exemplary embodiments.

One of the purposes of the present invention is achieved by a medical pendant arm system, the medical pendant arm system comprises:
an anchor plate, wherein the anchor plate is used for fixing to the roof structure in the installation space of the medical pendant arm system;
a first rotation shaft, wherein the first rotation shaft comprises a shaft outer ring, a shaft inner ring and a ball bearing; the ball bearing is disposed between the shaft outer ring and the shaft inner ring, the lower portion of the shaft outer ring forms a rectangular flange, the upper portion of the shaft inner ring forms a circular flange, wherein the circular flange of the first rotation shaft is fixed directly to the anchor plate or fixed to the anchor plate by means of an extension column; and
a first arm, wherein the first arm, in the vicinity of one end portion thereof, is fixed to the rectangular flange of the first rotation shaft, wherein the medical pendant arm system also comprises a rotation shaft cover provided outside the first rotation shaft and the rotation shaft cover is provided with a circular groove set between an upper rotation shaft cover and a lower rotation shaft cover thereof, wherein the circular groove is used for installing a circular brake indicator lamp.

According to the technical solutions described above, the medical pendant arm system of the present invention can have the following beneficial technical effects: compact overall structure, simple assembly and short assembly time, and relatively less parts, and an electromagnetic brake or an air-bag brake can be selectively installed.

Preferably, the medical pendant arm system further comprises:
a second rotation shaft, wherein the second rotation shaft comprises a shaft outer ring, a shaft inner ring and a ball bearing; the ball bearing is disposed between the shaft outer ring and the shaft inner ring, the lower portion of the shaft outer ring forms a rectangular flange, the upper portion of the shaft inner ring forms a circular flange, wherein the circular flange of the second rotation shaft is fixed in the vicinity of the other end portion of the first arm; and
a second arm, wherein the second arm, in the vicinity of one end portion thereof, is fixed to the rectangular flange of the second rotation shaft.

According to the technical solutions described above, the medical pendant arm system of the present invention can have the following beneficial technical effects: a dual arm system having compact overall structure is constituted, with simple assembly and short assembly time, and relatively less parts, and an electromagnetic brake or an air-bag brake can be selectively installed.

Preferably, the first rotation shaft and the second rotation shaft are identical rotation shafts.

According to the technical solutions described above, the medical pendant arm system of the present invention can have the following beneficial technical effects: modular design can be realized and its rotation shafts can be replaced each other, which save the production costs and the replacement costs.

Preferably, the end portions of the first arm or the second arm are downward bevelled.

According to the technical solutions described above, the medical pendant arm system of the present invention can have the following beneficial technical effects: the space occupancy of the medical pendant arm system is further reduced and the overall structural compactness is increased.

Preferably, an electromagnetic brake or an air-bag brake is mounted to the rectangular flange of the first rotation shaft or the rectangular flange of the second rotation shaft.

According to the technical solutions described above, the medical pendant arm system of the present invention can have the following beneficial technical effect: an electromagnetic brake or an air-bag brake can be selectively installed on the premise of no change for the structure of the rotation shafts.

As outlined above, the medical pendant arm system also comprises a rotation shaft cover provided outside the first rotation shaft or the second rotation shaft, and the rotation shaft cover is provided with a circular groove set between an upper rotation shaft cover and a lower rotation shaft cover thereof, which the circular groove is used for installing a circular brake indicator lamp.

According to the technical solutions described above, the medical pendant arm system of the present invention can have the following beneficial technical effect: a circular brake indicator lamp can be installed at an appropriate position to explicitly make lamplight indication when a brake of a certain rotation shaft releases.

Preferably, the first rotation shaft or the second rotation shaft also comprises a mechanical brake, and the periphery of the mechanical brake has threads for being screwed into a threaded hole of the rectangular flange of the first rotation shaft or the rectangular flange of the second rotation shaft.

According to the technical solutions described above, the medical pendant arm system of the present invention can have the following beneficial technical effect: a mechanical brake can be installed in a simple and effective manner to increase damping torque.

Preferably, the medical pendant arm system also comprises an upper lining board and a lower lining board respectively provided at the upper end and the lower end of the first rotation shaft or the second rotation shaft.

According to the technical solutions described above, the medical pendant arm system of the present invention can have the following beneficial technical effect: the contact stress at the junction of the rotation shaft and the arm is reduced.

Preferably, the upper surface of the first arm or the second arm is provided with an opening.

According to the technical solutions described above, the medical pendant arm system of the present invention can have the following beneficial technical effects:
on one aspect, it can facilitate the assembly of the gas pipes and lines, and on the other aspect, a top ambient illumination LED lamp can be selectively installed according to the requirements of customers.

Preferably, the first rotation shaft or the second rotation shaft also comprises a limit column and a limit block set outside the upper portion thereof.

According to the technical solutions described above, the medical pendant arm system of the present invention can have the following beneficial technical effect: the rotation range of the rotation shaft can be effectively limited.

Preferably, when the circular flange of the first rotation shaft is fixed to the anchor plate by means of the extension column, the extension column is composed of a cylindrical member having multiple extension column bores distributed along its circumference.

According to the technical solutions described above, the medical pendant arm system of the present invention can have the following beneficial technical effects: the extension column cover provided outside the extension column can be omitted, and the number of components is reduced on the premise that the good appearance and the cleanliness of the medical pendant arm system are ensured.

Preferably, the mechanical brake comprises a threaded portion located at its outside when installed, a friction head located inside when installed, and a disc spring located between the threaded portion and the friction head.

According to the technical solutions described above, the medical pendant arm system of the present invention can have the following beneficial technical effect: a preferable mode for a mechanical brake is provided, so that the damping torque can be increased in a simple and effective manner.

The above purposes of the present invention are also achieved by a rotation shaft used for a medical pendant arm system, the rotation shaft comprises a shaft outer ring, a shaft inner ring and a ball bearing, the ball bearing is disposed between the shaft outer ring and the shaft inner ring, the lower portion of the shaft outer ring forms a rectangular flange, and the upper portion of the shaft inner ring forms a circular flange.

According to the technical solutions described above, the rotation shaft used for the medical pendant arm system of the present invention can have the following beneficial technical effects: the overall structure of the entire medical pendant arm system is rendered to be compact, with simple assembly and short assembly time, and relatively less parts, and an electromagnetic brake or an air-bag brake can be selectively installed.

### Brief description of drawings

Figure 1 shows the front view for the medical pendant arm system of the present invention.
Figure 2 shows the partially cutaway three-dimensional view for the medical pendant arm system of the present invention.
Figure 3 shows the three-dimensional view for the rotation shaft used for the medical pendant arm system of the present invention, in which the upper and lower relationship of the rotation shaft is reversed (i.e., in contrast to the upper and lower relationship when it is installed in the medical pendant arm system) for clarity.
Figure 4 shows the partially cutaway three-dimensional view for the rotation shaft used for the medical pendant arm system of the present invention, in which the upper and lower relationship of the rotation shaft is reversed for clarity.
Figure 5 shows the section view for the rotation shaft used for the medical pendant arm system of the present invention, in which the upper and lower relationship of the rotation shaft is reversed for clarity.
Figure 6 shows another embodiment for the extension column used for the medical pendant arm system, in which figure 6(a) shows the partially cutaway schematic of the extension column when it is installed in the medical pendant arm system, and figure 6(b) separately shows the three-dimensional view for the extension column.
Figure 7(a) shows the front view for the mechanical brake used for the medical pendant arm system, and figure 7(b) shows the three-dimensional view for the mechanical brake used for the medical pendant arm system.

### Sign list for drawings:

10. anchor plate;
11. first rotation shaft;
12. first arm;
20. extension column;
20. extension column';
21. second rotation shaft;
22. second arm;
31. upper rotation shaft cover;
32. lower rotation shaft cover;
41. upper lining board;
42. lower lining board;
51. opening;
52. opening;
100. medical pendant arm system;
111. shaft outer ring;
112. shaft inner ring;
113. ball bearing;
114. rectangular flange;
115. circular flange;
116. lower sealing ring;
117. upper sealing ring;
118. mechanical brake;
119. limit column;
120. limit block;
121. cushion;
201. extension column bore;
1181. friction head;
1182. disc spring;
1183. threaded portion.

### Detailed description

The present invention is further described in connection with drawings and particular embodiments as follows and elaborated in more detail in the following description in order to fully understand the present invention, but it is evident that the present invention can be implemented in many other ways which are different from those described herein; generalization and deduction can be made by a skilled in the art without departing from the connotation of the invention according to practical application, and therefore the protective scope of the present invention should not be limited by the specific content of embodiments of the present invention herein.

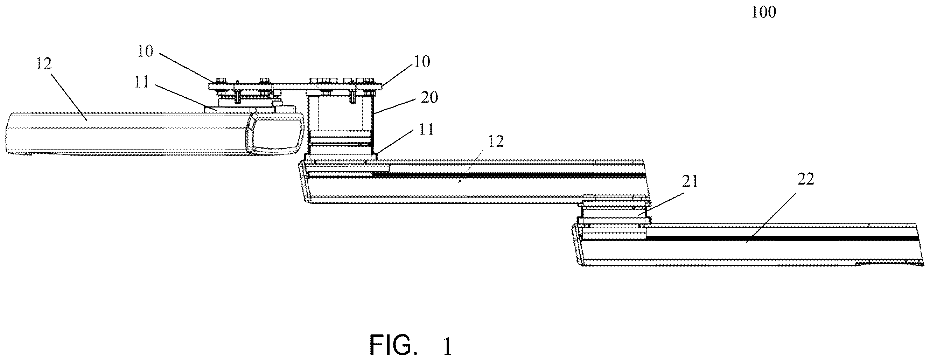

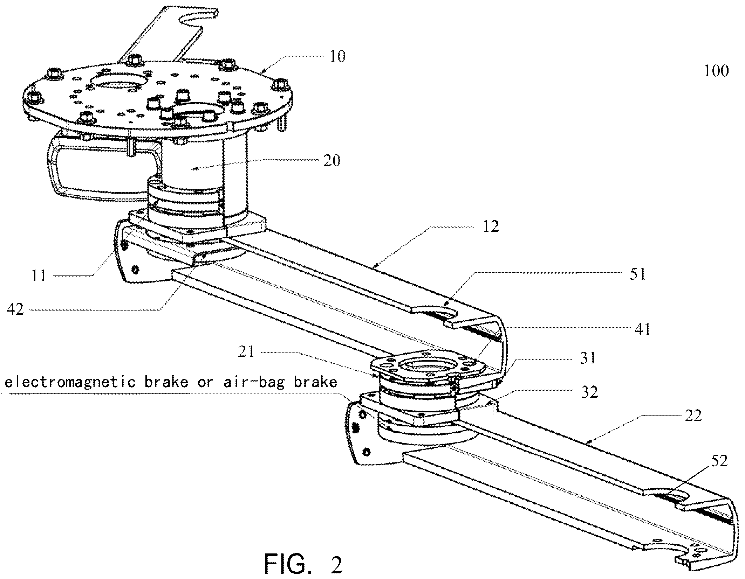

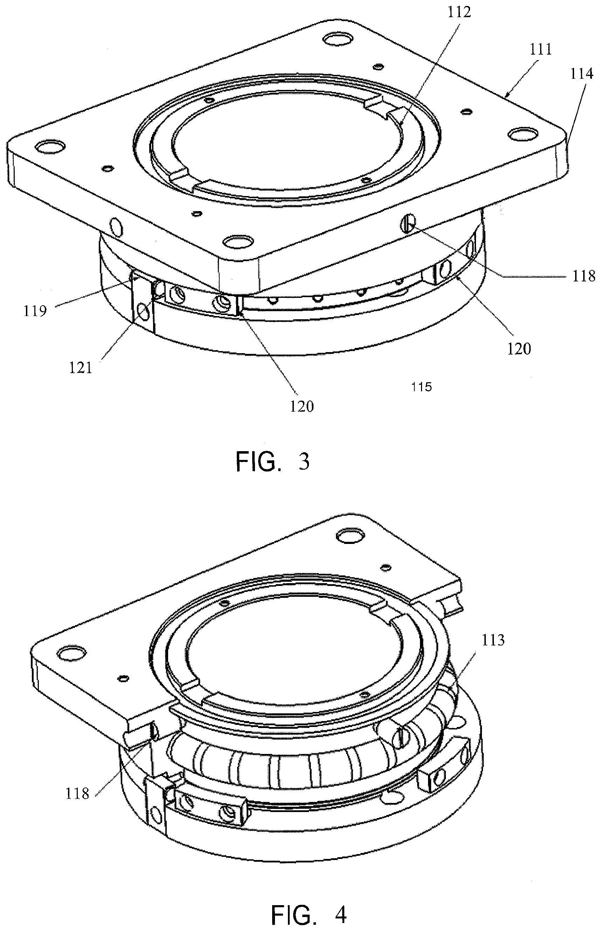

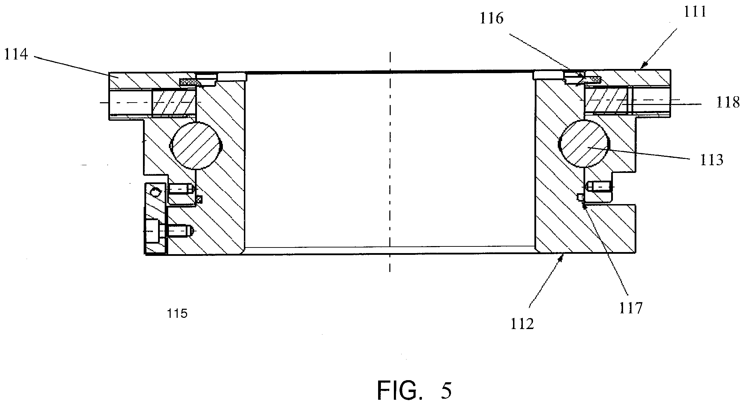

Figure 1 shows the front view for the medical pendant arm system 100 of the present invention. Figure 2 shows the partially cutaway three-dimensional view for the medical pendant arm system 100 of the present invention. Figure 3 shows the three-dimensional view for the rotation shafts 11 and 21 used for the medical pendant arm system 100 of the present invention, in which the upper and lower relationship of the rotation shaft is reversed (i.e., in contrast to the upper and lower relationship when it is installed in the medical pendant arm system 100) for clarity. Figure 4 shows the partially cutaway three-dimensional view for the rotation shafts 11 and 21 used for the medical pendant arm system 100 of the present invention, in which the upper and lower relationship of the rotation shaft is reversed for clarity. Figure 5 shows the section view for the rotation shafts 11 and 21 used for the medical pendant arm system 100 of the present invention, in which the upper and lower relationship of the rotation shaft is reversed for clarity.

The medical pendant arm system 100 of the present invention comprises:
an anchor plate 10, wherein the anchor plate 10 is used for fixing to the roof structure in the installation space of the medical pendant arm system, the top structure is, for example, the ceilings of the operating room and the intensive care unit in a hospital;
a first rotation shaft 11, wherein the first rotation shaft 11 comprises a shaft outer ring 111, a shaft inner ring 112 and a ball bearing 113, the ball bearing 113 is provided between the shaft outer ring 111 and the shaft inner ring 112, the lower portion of the shaft outer ring 111 forms a rectangular flange 114, the upper portion of the shaft inner ring 112 forms a circular flange 115, wherein the circular flange 115 of the first rotation shaft 11 is fixed directly to the anchor plate 10 or fixed to the anchor plate 10 by means of an extension column 20; and
a first arm 12, wherein the first arm 12, in the vicinity of one end portion thereof, is fixed to the rectangular flange 114 of the first rotation shaft 11.

In this way, the medical pendant arm system of the present invention can realize modular design, has a compact overall structure, is easy to assemble with a short assembly time, and has relatively less parts, and an electromagnetic brake or an air-bag brake can be selectively installed.

Note that, "upper", "lower", "front", "rear", "left", "right" and the like used herein are only exemplary directions defined to facilitate the description of the invention. For example, as shown in figure 1, the direction of the top side in the paper is "upper", and the direction of the bottom side in the paper is "lower", however, as shown in figure 5, the upper and lower relationship is reversed for clarity, that is, the direction of the top side in the paper is "lower", and the direction of the bottom side in the paper is "upper". Of course, those skilled in the art on the basis of the present invention can understood that "upper", "lower", "front", "rear", "left", "right" and other directions can also be defined in other ways.

Preferably, the medical pendant arm system 100 of the present invention further comprises:
a second rotation shaft 21, wherein the second rotation shaft comprises a shaft outer ring 111, a shaft inner ring 112 and a ball bearing 113, the ball bearing 113 is provided between the shaft outer ring 111 and the shaft inner ring 112, the lower portion of the shaft outer ring 111 forms a rectangular flange 114, the upper portion of the shaft inner ring 112 forms a circular flange 115,wherein the circular flange 115 of the second rotation shaft 21 is fixed in the vicinity of the other end portion of the first arm 12; and
A second arm 22, wherein the second arm 22, in the vicinity of one end portion thereof, is fixed to the rectangular flange 114 of the second rotation shaft 21.

In this way, a dual-arm system having compact overall structure is constituted, especially the distance between the arms is effectively shortened, with simple assembly and short assembly time, and relatively less parts, and an electromagnetic brake or an air-bag brake can be selectively installed.

Preferably, the first rotation shaft 11 and the second rotation shaft 21 are identical rotation shafts. Furthermore, preferably the first arm 12 and the second arm 22 are identical arms.

In this way, the medical pendant arm system of the present invention can realize modular design and its main components (rotation shafts and/or arms) can be replaced each other, which save the production costs and the replacement costs.

Although the examples given above only describe the circumstances with respect one rotation shaft and one arm or two rotation shafts and two arms, a person skilled in the art on the basis of the present invention should understand that not less than three rotation shafts and not less than three arms can also be used. Such variation also falls into the protection scope of the present invention.

Take the circumstance of dual pendant as an example, the circular flange of the rotation shaft 11 of one of the medical pendant (the medical pendant on the left in figure 1) is directly connected and fixed to the anchor plate 10, in the other medical pendant (the medical pendant on the right in figure 1), in order to avoid mutual interference, it is needed to install an extension column 20 on the circular flange of the rotation shaft 11. That is to say, the circular flange of the rotation shaft 11 of the medical pendant on the right in figure 1 is fixed to the anchor plate 10 by means of the extension column 20.

In order to ensure that the height size of the extension column 20 is small enough, it is preferable that the end portion of the arms 12 and 22 are downward bevelled. In this way, the space occupancy of the medical pendant arm system can be further reduced and the overall structural compactness is increased.

Furthermore, in order to ensure that the center distance between the rotation shafts 11 and 11 of the two medical pendants is less than 300 mm, the rotation shafts are designed into a layout that the circular flange is at top and the rectangular flange is at bottom (i.e., the lower portion of the shaft outer ring forms the rectangular flange, and the upper portion of the shaft inner ring forms the circular flange). In this way, not only the center distance between the rotation shafts of the two medical pendants can be shortened, but also the appearance of the extension column cover can be more concise and elegant.

The lower rectangular flanges of the rotation shafts 11 and 21 are designed into rectangular shape, the purpose of which is to install an electromagnetic brake (for example, of 120 Nm brake torque) at the lower ends of the rotation shafts 11 and 21, or to install an air-bag brake on the premise of no change of structure of the rotation shafts according to the requirements of the customers. That is to say, preferably, the rectangular flanges of the rotation shafts can be used for installing an electromagnetic brake or an air-bag brake. In particular, for example, a snap port for the electromagnetic brake and a backing board-fixing hole for the air-bag brake can be provided on the shaft inner ring 111, and an electromagnetic brake-fixing hole or an air-bag brake-fixing hole can be provided on the shaft outer ring 112.

The medical pendant arm system according to the invention also comprises a rotation shaft covers 31 and 32 provided outside the rotation shafts 11 and 21, and the rotation shaft covers are provided with a circular groove set between the upper rotation shaft cover 31 and the lower rotation shaft cover 32 thereof, which the circular groove is used for installing a circular brake indicator lamp. The circular brake indicator lamp will light up when a brake of the rotation shafts 11 and 21 releases.

Preferably, the end covers of the arms 12 and 22 are composed of two portions: the end cover housing and the end cover-fixing board, in this case, when the end covers are installed, the end cover-fixing board is firstly fixed by means of several (e.g., two) screws and several threaded holes inside the arms 12 and 22, then the end cover housing is directly pushed into the corresponding hole in the end cover-fixing board. In this way, the time for the assembly and disassembly of the arm end covers is shortened, and the complexity for the installation is reduced.

Preferably, as shown in figures 3-5, the rotation shafts 11 and 21 also comprise a mechanical brake 118, the periphery of the mechanical brake 118 has threads for being screwed into the threaded hole of the rectangular flange 114 of the rotation shafts 11 and 21. The number of mechanical brakes 118 can be increased according to the space, thus increasing the damping torque. For example, in the examples shown in figures 3-5, four mechanical brakes 118 are employed.

Preferably, in order to reduce the contact stress at the junction between the rotation shafts 11 and 21 and the arms 12 and 22 (aluminium section in general) and increase the connecting strength, the medical pendant arm system also comprises an upper lining board 41 and a lower lining board 42 disposed at the upper end and the lower end of the rotation shaft, respectively.

Preferably, the upper surfaces of the arms 12 and 22 are provided with openings 51 and 52. In this way, on one aspect, it can facilitate the assembly of the gas pipes and lines, and on the other aspect, a top ambient illumination LED lamp can be selectively installed according to the requirements of customers.

Preferably, as shown in figures 3-5, the first rotation shafts 11 and 21 also comprise a limit column 119 and a limit block 120 set outside the upper portion thereof. For example, the upper circular flange of the rotation shaft 11 is totally provided with 6 round holes which are uniformly distributed, but the rotation shaft 21 only need to use 4 holes based on the overturning moment difference supported by them.

Preferably, as shown in figure 3, on the area of the limit column 119 to be contacted with the limit block 120, there is further provided with a cushion 121.

Preferably, as shown in figure 5, the rotation shafts 11 and 21 also comprise an upper sealing ring 117 and a lower sealing ring 116, the upper sealing ring 117 is provided between the upper inside of the shaft outer ring 111 and the upper outside of the shaft inner ring 112, and the lower sealing ring 116 is provided between the lower inside of the shaft outer ring 111 and the lower outside of the shaft inner ring 112.

As shown in figure 2, when the circular flange 115 of the first rotation shaft 11 is fixed to the anchor plate 10 by means of the extension column 20, an extension column cover can be provided outside the extension column 20 for the purpose of the good appearance and cleanliness of the medical pendant arm system.

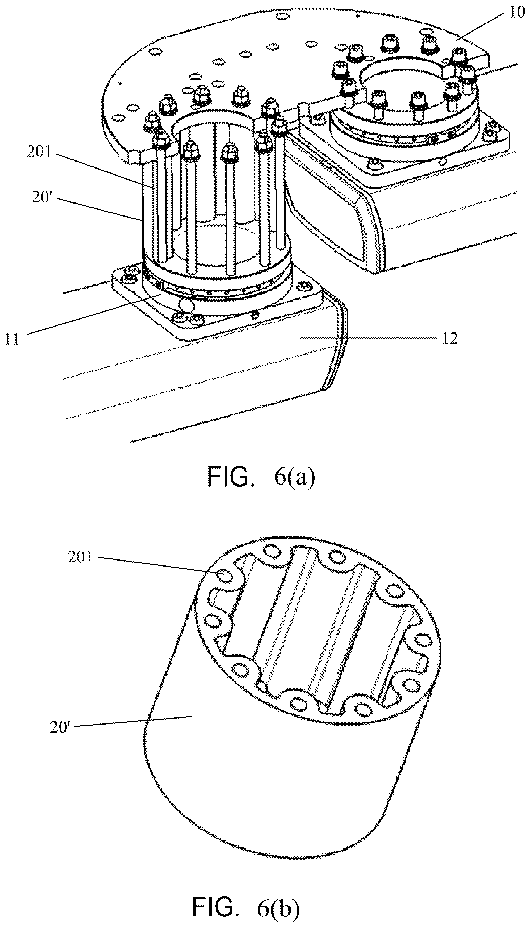

Figure 6 shows another embodiment for the extension column used for the medical pendant arm system, in which figure 6(a) shows the partially cutaway schematic of the extension column when it is installed in the medical pendant arm system, and figure 6(b) separately shows the three-dimensional view for the extension column.

The extension column 20' is composed of a cylindrical member having multiple extension column bores 201 distributed along its circumference. When the circular flange 115 of the first rotation shaft 11 is fixed to the anchor plate 10 by means of the extension column 20', multiple threaded rods pass through multiple extension column bores 201, respectively, and their lower ends are connected to the threaded holes located in the first rotation shaft 11, and their upper ends pass through the unthreaded holes (non-threaded holes) in the anchor plate 10 and tightened via the nuts above the anchor plate 10. The extension column 20' can be subjected to outer surface spray coating. In this way, the extension column cover provided outside the extension column is omitted.

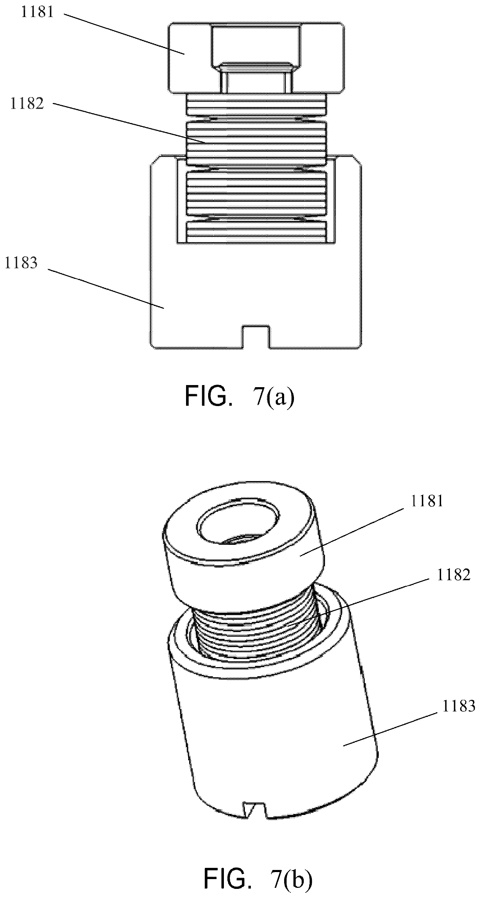

Figure 7(a) shows the front view for the mechanical brake used for the medical pendant arm system, and figure 7(b) shows the three-dimensional view for the mechanical brake used for the medical pendant arm system.

The mechanical brake 118 comprises a threaded portion 1183 located outside (i.e., the side away from the shaft inner ring) when it is installed (i.e., when it is installed in the rotation shaft), a friction head 1181 located inside (i.e., the side toward the shaft inner ring) when it is installed, and a disc spring 1182 located between the threaded portion 1183 and the friction head 1181. When the mechanical brake 118 is to be installed in the rotation shaft, a screwdriver can be used to screw the mechanical brake 118 into the rotation shaft, when a certain torque is achieved, the friction head 1181, which is suffered from the press of the disc spring 1182, presses tightly against the outer cylindrical surface of the shaft inner ring 111.

The present invention has been exemplarily described above in connection with the figures, although the specific implementations of the present invention are not limited to the above embodiments, but by the scope of the appended claims.

## Claims

1. A medical pendant arm system (100), wherein the medical pendant arm system (100) comprises:
an anchor plate (10), wherein the anchor plate (10) is configured to fix to a roof structure in an installation space of the medical pendant arm system (100);
a first rotation shaft (11), wherein the first rotation shaft (11) comprises a shaft outer ring (111), a shaft inner ring (112) and a ball bearing (113); the ball bearing (113) is disposed between the shaft outer ring (111) and the shaft inner ring (112),
wherein the lower portion of the shaft outer ring (111) forms a rectangular flange (114), the upper portion of the shaft inner ring (112) forms a circular flange (115), wherein the circular flange (115) of the first rotation shaft (11) is fixed directly to the anchor plate (10) or fixed to the anchor plate (10) by means of an extension column (20); and
a first arm (12), wherein the first arm (12), in the vicinity of one end portion thereof, is fixed to the rectangular flange (114) of the first rotation shaft (11), wherein the medical pendant arm system also comprises a rotation shaft cover provided outside the first rotation shaft and the rotation shaft cover is provided with a circular groove set between an upper rotation shaft cover and a lower rotation shaft cover thereof, wherein the circular groove is configured to receive a circular brake indicator lamp.

2. The medical pendant arm system of claim 1, wherein the medical pendant arm system further comprises:
a second rotation shaft, wherein the second rotation shaft comprises a shaft outer ring, a shaft inner ring and a ball bearing; the ball bearing is disposed between the shaft outer ring and the shaft inner ring, the lower portion of the shaft outer ring forms a rectangular flange, the upper portion of the shaft inner ring forms a circular flange, wherein the circular flange of the second rotation shaft is fixed in the vicinity of the other end portion of the first arm; and
a second arm, wherein the second arm, in the vicinity of one end portion thereof, is fixed to the rectangular flange of the second rotation shaft.

3. The medical pendant arm system of claim 2, wherein the first rotation shaft and the second rotation shaft are identical rotation shafts.

4. The medical pendant arm system of claim 1 or 2, wherein the end portion of the first arm or the second arm is downward bevelled.

5. The medical pendant arm system of claim 1 or 2, wherein an electromagnetic brake or an air-bag brake is mounted to the rectangular flange of the first rotation shaft or the rectangular flange of the second rotation shaft.

6. The medical pendant arm system of claim 1 or 2, wherein the first rotation shaft or the second rotation shaft also comprises a mechanical brake, and the periphery of the mechanical brake has threads for being screwed into a threaded hole of the rectangular flange of the first rotation shaft or the rectangular flange of the second rotation shaft.

7. The medical pendant arm system of claim 1 or 2, wherein the medical pendant arm system also comprises an upper lining board and a lower lining board respectively provided at the upper end and the lower end of the first rotation shaft or the second rotation shaft.

8. The medical pendant arm system of claim 1 or 2, wherein the upper surface of the first arm or the second arm is provided with an opening.

9. The medical pendant arm system of claim 1, wherein when the circular flange of the first rotation shaft is fixed to the anchor plate by means of the extension column, the extension column is composed of a cylindrical member having multiple extension column bores distributed along circumference.

10. The medical pendant arm system of claim 6, wherein the mechanical brake comprises a threaded portion located at its outside when installed, a friction head located at is inside when installed, and a disc spring located between the threaded portion and the friction head.

## Patentansprüche

1. Medizinisches Schwenkarmsystem (100), wobei das medizinische Schwenkarmsystem (100) aufweist:
eine Verankerungsplatte (10), wobei die Verankerungsplatte (10) so ausgebildet ist, dass sie an einer Deckenstruktur in einem Installationsraum des medizinischen Schwenkarmsystems (100) befestigbar ist,
einen ersten Rotationsschaft (11), wobei der erste Rotationsschaft (11) einen Schaftaußenring (111), einen Schaftinnenring (112) und ein Kugellager (113) aufweist, wobei das Kugellager (113) zwischen dem Schaftaußenring (111) und dem Schaftinnenring (112) angeordnet ist, wobei der untere Abschnitt des Schaftaußenrings (111) einen rechteckigen Flansch (114) bildet, wobei der obere Abschnitt des Schaftinnenrings (112) einen kreisförmigen Flansch (115) bildet, wobei der kreisförmige Flansch (115) des ersten Rotationsschafts (11) direkt an der Verankerungsplatte (10) befestigt ist oder an der Verankerungsplatte (10) mit Hilfe einer Erweiterungssäule (20) befestigt ist, und
einen ersten Arm (12), wobei der erste Arm (12) in der Nähe des einen Endabschnitts davon an dem rechteckigen Flansch (114) des ersten Rotationsschafts (11) befestigt ist, wobei das medizinische Schwenkarmsystem auch eine Rotationsschaftabdeckung aufweist, welche außerhalb des ersten Rotationsschafts angeordnet ist, und wobei die Rotationsschaftabdeckung eine kreisförmige Nut aufweist, welche zwischen einer oberen Rotationsschaftabdeckung und einer unteren Rotationsschaftabdeckung davon angeordnet ist, wobei die kreisförmige Nut dafür ausgebildet ist, eine kreisförmige Bremsanzeigenlampe aufzunehmen.

2. Medizinisches Schwenkarmsystem nach Anspruch 1, wobei das medizinische Schwenkarmsystem weiterhin aufweist:
einen zweiten Rotationsschaft, wobei der zweite Rotationsschaft einen Schaftaußenring, einen Schaftinnenring und ein Kugellager aufweist, wobei das Kugellager zwischen dem Schaftaußenring und dem Schaftinnenring angeordnet ist, wobei der untere Abschnitt des Schaftaußenrings einen rechteckigen Flansch bildet, wobei der obere Abschnitt des Schaftinnenrings einen kreisförmigen Flansch bildet, wobei der kreisförmige Flansch des zweiten Rotationsschafts in der Nähe des anderen Endabschnitts des ersten Arms befestigt ist, und
einen zweiten Arm, wobei der zweite Arm in der Nähe des einen Endabschnitts davon an dem rechteckigen Flansch des zweiten Rotationsschafts befestigt ist.

3. Medizinisches Schwenkarmsystem nach Anspruch 2, wobei der erste Rotationsschaft und der zweite Rotationsschaft identische Rotationsschäfte sind.

4. Medizinisches Schwenkarmsystem nach Anspruch 1 oder 2, wobei der Endabschnitt des ersten Arms oder des zweiten Arms nach unten hin abgeschrägt ist.

5. Medizinisches Schwenkarmsystem nach Anspruch 1 oder 2, wobei eine elektromagnetische Bremse oder eine Airbag-Bremse auf dem rechteckigen Flansch des ersten Rotationsschafts oder dem rechteckigen Flansch des zweiten Rotationsschafts befestigt ist.

6. Medizinisches Schwenkarmsystem nach Anspruch 1 oder 2, wobei der erste Rotationsschaft oder der zweite Rotationsschaft auch eine mechanische Bremse aufweist, und die Peripherie der mechanischen Bremse Gewinde zum Einschrauben in eine Gewindebohrung des rechteckigen Flansches des ersten Rotationsschafts oder des rechteckigen Flansches des zweiten Rotationsschafts aufweist.

7. Medizinisches Schwenkarmsystem nach Anspruch 1 oder 2, wobei das medizinische Schwenkarmsystem auch eine obere Auskleidungsplatte und eine untere Auskleidungsplatte aufweist, welche jeweils an dem oberen Ende und dem unteren Ende des ersten Rotationsschafts oder des zweiten Rotationsschafts angeordnet sind.

8. Medizinisches Schwenkarmsystem nach Anspruch 1 oder 2, wobei die obere Fläche des ersten Arms oder des zweiten Arms mit einer Öffnung versehen ist.

9. Medizinisches Schwenkarmsystem nach Anspruch 1, wobei wenn der kreisförmige Flansch des ersten Rotationsschafts mit Hilfe der Erweiterungssäule an der Verankerungsplatte befestigt ist, die Erweiterungssäule aus einem zylindrischen Element mit mehreren Erweiterungssäulenbohrlöchern besteht, welche entlang des Umfangs verteilt sind.

10. Medizinisches Schwenkarmsystem nach Anspruch 6, wobei die mechanische Bremse einen Gewindeabschnitt, welcher an der Außenseite angeordnet ist, wenn er installiert ist, einen Reibungskopf, welcher an der Innenseite angeordnet ist, wenn er installiert ist, und eine Tellerfeder, welche zwischen dem Gewindeabschnitt und dem Reibungskopf angeordnet ist, aufweist.

## Revendications

1. Système de bras suspendu médical (100), dans lequel le système de bras suspendu médical (100) comprend :
une plaque d'ancrage (10), dans lequel la plaque d'ancrage (10) est configurée pour se fixer à une structure de toit dans un espace d'installation du système de bras suspendu médical (100) ;
un premier arbre de rotation (11), dans lequel le premier arbre de rotation (11) comprend une bague extérieure d'arbre (111), une bague intérieure d'arbre (112) et un roulement à billes (113) ; le roulement à billes (113) est disposé entre la bague extérieure d'arbre (111) et la bague intérieure d'arbre (112),
dans lequel la partie inférieure de la bague extérieure d'arbre (111) forme un rebord rectangulaire (114), la partie supérieure de la bague intérieure d'arbre (112) forme une rebord circulaire (115), dans lequel le rebord circulaire (115) du premier arbre de rotation (11) est fixé directement à la plaque d'ancrage (10) ou fixé à la plaque d'ancrage (10) au moyen d'une colonne d'extension (20) ; et
un premier bras (12), dans lequel le premier bras (12), au voisinage d'une première partie d'extrémité de celui-ci, est fixé au rebord rectangulaire (114) du premier arbre de rotation (11), dans lequel le système de bras suspendu médical comprend également un couvercle d'arbre de rotation prévu à l'extérieur du premier arbre de rotation, et le couvercle d'arbre de rotation est pourvu d'une rainure circulaire établie entre un couvercle d'arbre de rotation supérieur et un couvercle d'arbre de rotation inférieur de celui-ci, dans lequel la rainure circulaire est configurée pour recevoir un témoin lumineux de frein circulaire.

2. Système de bras suspendu médical selon la revendication 1, dans lequel le système de bras suspendu médical comprend en outre :
un second arbre de rotation, dans lequel le second arbre de rotation comprend une bague extérieure d'arbre, une bague intérieure d'arbre et un roulement à billes ; le roulement à billes est disposé entre la bague extérieure d'arbre et la bague intérieure d'arbre, la partie inférieure de la bague extérieure d'arbre forme un rebord rectangulaire, la partie supérieure de la bague intérieure d'arbre forme un rebord circulaire, dans lequel le rebord circulaire du second arbre de rotation est fixé au voisinage de l'autre partie d'extrémité du premier bras ; et
un second bras, dans lequel le second bras, au voisinage d'une première partie d'extrémité de celui-ci, est fixé au rebord rectangulaire du second arbre de rotation.

3. Système de bras suspendu médical selon la revendication 2, dans lequel le premier arbre de rotation et le second arbre de rotation sont des arbres de rotation identiques.

4. Système de bras suspendu médical selon la revendication 1 ou 2, dans lequel la partie d'extrémité du premier bras ou du second bras est biseautée vers le bas.

5. Système de bras suspendu médical selon la revendication 1 ou 2, dans lequel un frein électromagnétique ou un frein à coussin gonflable est monté sur le rebord rectangulaire du premier arbre de rotation ou sur le rebord rectangulaire du second arbre de rotation.

6. Système de bras suspendu médical selon la revendication 1 ou 2, dans lequel le premier arbre de rotation ou le second arbre de rotation comprend également un frein mécanique, et la périphérie du frein mécanique a des filets pour être vissée dans un trou fileté du rebord rectangulaire du premier arbre de rotation ou du rebord rectangulaire du second arbre de rotation.

7. Système de bras suspendu médical selon la revendication 1 ou 2, dans lequel le système de bras suspendu médical comprend également un panneau de revêtement supérieur et un panneau de revêtement inférieur respectivement prévus à l'extrémité supérieure et à l'extrémité inférieure du premier arbre de rotation ou du second arbre de rotation.

8. Système de bras suspendu médical selon la revendication 1 ou 2, dans lequel la surface supérieure du premier bras ou du second bras est pourvue d'une ouverture.

9. Système de bras suspendu médical selon la revendication 1, dans lequel lorsque le rebord circulaire du premier arbre de rotation est fixé à la plaque d'ancrage au moyen de la colonne d'extension, la colonne d'extension est composée d'un élément cylindrique ayant de multiples alésages de colonne d'extension répartis le long de la circonférence.

10. Système de bras suspendu médical selon la revendication 6, dans lequel le frein mécanique comprend une partie filetée située au niveau de son extérieur après installation, une tête de friction située au niveau de son intérieur après installation, et un ressort à disque situé entre la partie filetée et la tête de friction.
